# EUROPEAN PATENT APPLICATION

(11) **EP 3 797 743 A2**
(43) Date of publication of application: **31.03.2021**
(21) Application number: 20201567.3
(22) Date of filing: 09.05.2011
(51) Int. Cl.: A61F 9/008

(54) **SYSTEM AND METHOD FOR TREATING AN EYE**

(30) Priority: 10.05.2010 US 33299410 P
(62) Divisional of application: 11724805.4
(71) Applicant: Ramot at Tel Aviv University, Ltd., 61392 Tel Aviv (IL); Tel HaShomer Medical Research Infrastructure and Services Ltd., 52 621 Tel Hashomer (IL)
(72) Inventor: Belkin, Michael, 54044 Givat Schmuel (IL); Goldenfeld, Mordechai, 69494 Tel Aviv (IL)
(74) Representative: Beck Greener LLP

(57) **Abstract**

The invention provides a device for delivering electromagnetic radiation to a limbal area of an eye that transforms one or more beams of electromagnetic radiation that are incident on a first side into one or more emitted beams of the electromagnetic radiation where the one or more beams are arrayed in a cylindrical array, an array of one or more beams each beam having a cross sectional shape of a circular arc or an array comprising a beam having an annular cross section. The invention also provides a system for delivering electromagnetic radiation to the limbal area of an eye that includes one or more devices of the invention and a source of electromagnetic radiation. The system may be used in the treatment of glaucoma.

## Description

### FIELD OF THE INVENTION

This invention relates to medical devices, and in particular to such devices for use in ophthalmology.

### BACKGROUND OF THE INVENTION

The following documents are considered to be relevant for an understanding of the background of the invention:
Barkana, Y and Belkin M., Selective Laser Trabeculoplasty, Survey of Ophthalmology 52:634-653, 2007.

US Patent No. 6,698,886 to Pollack et al.

US Patent 5,479,222 to Volk.

Under normal circumstances, aqueous humor is secreted into the posterior chamber of the eye, and then circulates through the pupil into the anterior chamber where it passes through the trabecular meshwork, before being secreted from the eye. In most forms of glaucoma, the flow of the aqueous humor through the trabecular meshwork is impeded, preventing adequate drainage of the aqueous humor from the eye. This leads to a rise in the intraocular pressure, a state which may cause damage to the eye and lead to progressive blindness. One method to treat or prevent this from occurring is to alter some of the trabecular meshwork in order to improve the flow of aqueous humor through the trabecular meshwork..

A common method of enhancing the flow of aqueous humor through the trabecular meshwork is laser trabeculoplasty (LTP) which consists of the application of laser energy to the trabecular meshwork. There are a several types of LTP, such as selective laser trabeculoplasty (SLT) argon laser trabeculoplasty (ALT), diode laser trabeculoplasty (DLT), micropulse laser trabeculoplasty (MLT), and titanium:sapphire laser trabeculoplasty (TLT). The various types of LTP differ in the wavelength and other characteristics of the laser beam. SLT, for example, utilizes a Q-switched 532 Nd:YAG laser which selectively targets melanin-containing cells within the trabecular meshwork. (Barkana, Y et al) While the entire mechanism of action has not been completely elucidated, it is believed that laser-stimulated melanin-containing cells release cytokines which attract other cell types to the trabecular meshwork that increase its permeability. Unlike older versions of LTP, such as ALT, selective laser trabeculoplasty does not require precise targeting since the wavelength and energy of the light used selectively targets the melanine containing cells within the meshwork. The surrounding cells are not heated or destroyed. Thus, the fluid outflow is improved without damaging the trabecular meshwork. SLT has been used to treat primary open angle glaucoma, intraocular hypertension, normal tension glaucoma, aphakic (glaucoma in patients without a natural lens in their eye), pseudophakic glaucoma (glaucoma in patients without an artificial lens in their eye pigmentary, chronic angle closure glaucoma and juvenile glaucoma. SLT has also been successfully used to treat pressure increases in the eye caused by certain medications.

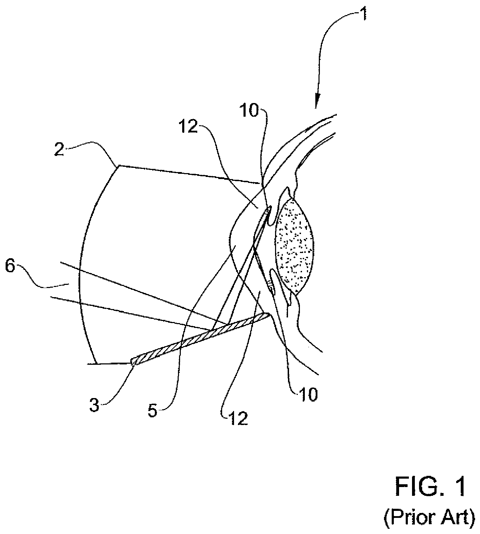

Fig. 1 shows the treatment of an eye **1** by LTP. Eye drops are first placed in the eye to provide surface anesthesia and to prepare the eye for the procedure. The trabecular meshwork **10** is situated around the angle of the anterior chamber of the eye and is not directly observable because it is obscured by the limbal area **12.** A gonioscopic contact lens **2** which includes a mirror **3** is applied to the eye **1** to direct a laser beam **6** through the cornea **5** to the trabecular meshwork **10** underneath the limbal area **12.** Typically, between 180° to 360° of the anterior chamber angle is irradiated by rotating the gonioscopic contact lens **12** after each laser pulse. About 100 laser pulses of a few nanoseconds duration and about 0.6 to 200 mJ of energy are delivered to the trabecular meshwork.

US Patent 5,479,222 to Volk discloses a gonioscopic lens system comprising at least two lenses. At least one of the lenses includes an aspheric surface of revolution. The lenses are positioned adjacent one another in a housing, such that the refractive properties of each are combined to converge light from an illumination light source to the entrance pupil of the patient's eye to illuminate the fundus. The lens system is designed for use with an associated ophthalmoscopic lens, enabling selective modification of the optical characteristics of the ophthalmoscopic lens system in a predetermined manner.

US Patent No. 6,698,886 to Pollack et al discloses an iridotomy and trabeculoplasty goniolaser lens having a contact lens element, a planar mirror offset from the optical axis of the contact lens element and first and second button lenses mounted on the anterior surface of the contact lens element. Magnification, curvature and location of the button lenses are chosen so as to provide the ability to simultaneously deliver laser energy to the iris of a patient's eye along a first optical path offset from the optical axis of the contact lens element and to view the trabecular meshwork around the region where the laser energy was applied.

Irradiating the trabecular meshwork with a laser beam directed through the cornea, as shown in Fig. 1, is often not possible in cases of narrow or closed angle glaucoma which occurs when the iris of the eye approaches the cornea and thus narrows or eliminates the angle between the cornea and the iris. This is the most common type of glaucoma in Chinese and Indian people and hence the commonest form of glaucoma and blindness in the world. In these cases, the laser beam cannot reach the trabeculum meshwork to be irradiated.

### SUMMARY OF THE INVENTION

The present invention is based on the novel and unexpected finding that LTP can be performed by irradiating the trabecular meshwork through the limbal area, thus avoiding the need for a gonioscopic contact lens. The inventors have found that irradiating the trabecular meshwork directly through the limbal area can achieve results comparable to those obtained by prior art LTP methods that utilize a gonioscopic contact lens. The inventors have found, for example, that a 532 nm laser beam is capable of penetrating the 1 mm thick limbal area and reaches the trabecular tissue to be treated with an adequate intensity to enhance the flow of aqueous humor through the trabecular meshwork, and to cause a significant decrease in intraocular pressure.

Thus, for example, in one patient suffering from open angle glaucoma with pseudoexfoliation, following irradiation of the limbal area with a 532 nm Nd:YAG laser (total energy delivered to a single eye around 10 J), the intraocular pressure reduced within a day from 24 to 14 mmHg while the patient was using antihypertensive eye drops. In another patient suffering from open angle glaucoma with pseudoexfoliation, the intraocular pressure decreased from 24 mmHg to 12 mmHg a week after treatment with a reduction of antihypertensive eye drops from 3 to 2. In a patient with primary open angle glaucoma the intraocular pressure was reduced from 27 to 18 mm Hg following the treatment.

Thus, in one of its aspects, the present invention provides a device for directing a beam of electromagnetic radiation to one or more regions on the limbal area of an eye. Device of the invention comprises a thin plate having one or more apertures that are arrayed in the plate to overly locations around the limbal area. The apertures may have, for example, a circular cross section or the apertures may have an arched shape. The plate may be rotatable so that each aperture can be positioned over different locations of the sclerlal limbus. The device may be configured to be placed directly onto the eye being treated, or the device may be configured to be held a predetermined distance away from the eye.

In another of its aspects, the invention provides a system for treating an eye. The system of the invention comprises a source of electromagnetic radiation, a device of the invention for directing radiation generated by the source to one or more location on the sclera limbus of an eye. The electromagnetic radiation may have, a wavelength in the visible or near infrared range, between 514 and 810 nm, and may be, for example, a 532 Nd:YAG laser. The system may be configured to generated pulses of the electromagnetic radiation. In this case, the pulses may be between 0.1 to 3 nanosec in duration, and the fluence of a single pulse may be 0.84 to 10,000 J/cm². The total energy delivered to a single eye may be from 4 to 20 J.

The invention also provides an ensemble comprising two or more devices of the invention for delivering electromagnetic radiation to a limbal area of an eye. In the ensemble of the invention, any two or more devices producing a cylindrical array of emitted beams produce a cylindrical array of emitted beams having a different cross-sectional diameter. Similarly, two or more devices producing emitted beams having a cross sectional shape of a circular arc produces emitted beams having a cross sectional shape of a circular arc of a different diameter. Two or more devices producing an emitted beam having an annular cross section produces an emitted beam having an annular cross section of one or both of a different inner diameter or a different outer diameter.

The ensemble of the invention may be used when it is desirable to irradiate the limbal area of an eye in a procedure using a series of irradiations of different geometrical parameters. Thus, for example, the eye may first be irradiated with a either a cylindrical array of beams or beams having a cross sectional shape of a circular arc wherein the cylindrical array or the circular arcs have a relatively small diameter (e.g. 9 mm), and then irradiated one or more additional times, each time increasing the diameter. The final diameter may be around 13 mm. Similarly, the eye may be sequentially irradiated with a series of beams having an annular cross section where one or both of the inner diameter and the outer diameter increases each time.

In still another of its aspects, the present invention provides a method for treating an eye. The method of the invention comprises directing electromagnetic radiation to one or more location on the sclera limbus of an eye. The electromagnetic radiation may have, a wavelength in the visible or near infrared range, between 514 and 810 nm, and may be generated, for example, by a 532 Nd:YAG laser. The electromagnetic radiation may be delivered to the limbal area in pulses. In this case, the pulses may be between 0.1 to 3 nanosecond in duration, and the fluence of a single pulse may be 0.84 to 10,000 J/cm². The total energy delivered to a single eye may be from 4 to 20 J. The method of the invention may be carried out using the system of the invention.

The invention may be used in the treatment of narrow or closed angle glaucoma, since, in accordance with the invention, the beam of electromagnetic radiation is not directed through the angle.

Thus, in one of its aspects, the invention provides a device for delivering electromagnetic radiation to a limbal area of an eye, the device having a first side and a second side, and the device transforming one or more beams of electromagnetic radiation that are incident on the first side into one or more beams of the electromagnetic radiation that are emitted from the second side, wherein the one or more emitted beams are arrayed in an array selected from:
**(a)** an array of two or more emitted beams that are arrayed in a cylindrical array, the cylindrical array having a circular cross section;
**(b)** an array of one or more beams each beam having a cross sectional shape of a circular arc; and
**(c)** an array comprising a beam having an annular cross section.

In the device of the invention, when the emitted radiation comprises two or more emitted beams that are arrayed in a cylindrical array, the cylindrical array may have a circular cross section having a diameter from 9 to 13 mm. When the emitted radiation comprises an array of one or more beams having a cross sectional shape of a circular arc; the circular arc may have a diameter from 9 to 13 mm. When the emitted radiation comprises an array comprising a beam having an annular cross section, the annular cross section may have an inner diameter and an outer diameter, the average of the inner diameter and the outer diameter being from 9 to 13 mm.

When the emitted radiation comprises two or more emitted beams that are arrayed in a cylindrical array, the device may comprise an element opaque to the electromagnetic radiation having therein a circular array of apertures extending from a first face of the opaque element to a second face of the opaque element. The circular array of apertures may comprise, for example, at least 50 apertures. The opaque element may be rotatable about the center of the circular array. When the emitted radiation comprises an array of one or more beams having a cross sectional shape of a circular arc; the device may comprise an element opaque to the electromagnetic radiation having therein a circular array of apertures, each aperture having a cross sectional shape of a circular arc and each aperture extending from a first face of the opaque element to a second face of the opaque element. The opaque element may be rotatable about the center of the circular array. When the emitted radiation comprises two or more emitted beams that are arrayed in a cylindrical array, the device may comprise a cylindrical array of optic fibers. The cylindrical array of optic fibers may be embedded in an opaque element, with the cylindrical array of optic fibers extending from a first face of the opaque element to a second face of the opaque element. When the emitted radiation comprises a beam having an annular cross section, and the device may comprise a refractive or diffractive optical element. When the emitted radiation comprises a beam having an annular cross section, the device may comprise an ellipsoidal mirror.

The device of the invention may further comprise a concave surface adjacent to the second side of the device. The concave surface may conform to the surface of the eye to promote stabilization of the device on an eye.

In another of its aspects, the invention provides an ensemble comprising two or more devices for delivering electromagnetic radiation from the first source of electromagnetic radiation to a limbal area of an eye, each of the one or more devices having a first side and a second side, and each device transforming one or more beams of electromagnetic radiation that are incident on the first side into one or more beams of the electromagnetic radiation that are emitted from the second side, wherein the one or more emitted beams are arrayed in an array selected from:
(i) an array of two or more emitted beams that are arrayed in a cylindrical array, the cylindrical array having a circular cross section, wherein each of the one or more devices producing a cylindrical array of emitted beams produces a cylindrical array of emitted beams having a different cross-sectional diameter;
(ii) an array of one or more beams having a cross sectional shape of a circular arc wherein each of the one or more devices producing emitted beams having a cross sectional shape of a circular arc produces emitted beams having a cross sectional shape of a circular arc of a different diameter ; and
(iii) an array comprising a beam having an annular cross section, wherein each of the one or more devices producing an emitted beam having an annular cross section produces an emitted beam having an annular cross section of one or both of a different inner diameter or a different outer diameter.

The invention also provides a system for delivering electromagnetic radiation to a limbal area of an eye, comprising:
**(a)** a first source of electromagnetic radiation; and
**(b)** one or more devices for delivering electromagnetic radiation from the first source of electromagnetic radiation to a limbal area of an eye the device when the first source is optically coupled to the device, each of the one or more devices having a first side and a second side, and each device transforming one or more beams of electromagnetic radiation from the first source that are incident on the first side into one or more beams of the electromagnetic radiation that are emitted from the second side, wherein the one or more emitted beams are arrayed in an array selected from:
   (i) an array of two or more emitted beams that are arrayed in a cylindrical array, the cylindrical array having a circular cross section, wherein each of the one or more devices produces a cylindrical array of emitted beams having a different cross-sectional diameter;
   (ii) an array of one or more beams having a cross sectional shape of a circular arc wherein each of the one or more devices produces emitted beams having a cross sectional shape of a circular arc of a different diameter; and
   (iii) an array comprising a beam having an annular cross section, wherein each of the one or more devices produces emitted beams having an annular cross section of a different inner diameter or a different outer diameter.

In the system of the invention, the first source may be a laser. The laser may have, for example, a wavelength from 514 810 nm. The laser may be, for example, a 532 Nd:YAG laser.

The system may further comprise a processor configured to execute a predetermined regime of activation of the first source of electromagnetic radiation. The predetermined regime of activation of the source of electromagnetic radiation may comprises a series of pulses. The pulses may be between 0.1 to 3 nanoseconds in duration and the fluence of a single pulse may be from 0.84 to 10,000 J/cm2. The total energy delivered to a single eye may be from 4 to 20 J.

The system may further comprise a second source of electromagnetic radiation that produces a visible light beam, where the second source of radiation is configured to be optically coupled to the device of the system.

The invention also provides a method for delivering electromagnetic radiation to a limbal area of an eye, comprising
**(a)** delivering electromagnetic radiation to the limbal area of the eye wherein the delivered electromagnetic radiation is in the form of one or more beams, the beams being arrayed in an array selected from:
   (i) an array of two or more emitted beams that are arrayed in a cylindrical array, the cylindrical array having a circular cross section;
   (ii) an array of one or more beams having a cross sectional shape of a circular arc; and
   (iii) an array comprising a beam having an annular cross section.
The method may be used in the treatment of glaucoma.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** shows the beam path in SLT using a gonioscopy contact lens;
**Fig. 2** shows a device for directing electric magnetic radiation to one or more regions of a limbal area of an eye having a circular array of apertures, in accordance with one embodiment of the invention;
**Fig. 3** shows a device for directing electric magnetic radiation to one or more regions of a limbal area of an eye having an array or arc shaped apertures, in accordance with another embodiment of the invention;
**Fig. 4** shows a device for directing electric magnetic radiation to one or more regions of a limbal area of an eye having a circular array of apertures, where the array is rotatable;
**Fig. 5** shows a device for directing electric magnetic radiation to one or more regions of a limbal area of an eye that includes oor more optic fibers arranged in a cylinder; and
**Fig. 6** shows a system for delivering electromagnetic radiation to a limbal area of an eye in accordance with one embodiment of this aspect of the invention.

### DETAILED DESCRIPTION OF EMBODIMENTS

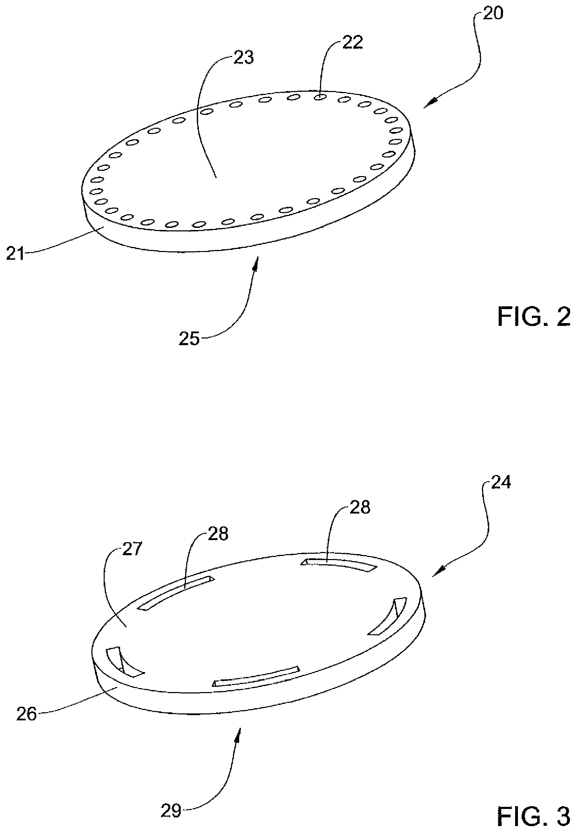

Fig. 2 shows a device **20** for directing electric magnetic radiation to one or more regions of a limbal area of an eye in accordance with one embodiment of the invention. The device **20** comprises a thin plate **21** that may be a circular disk. The plate **2** is provided with a plurality of small apertures **22** that may have a circular cross section. The plate **2** is formed from an opaque material such as metal. The apertures **22** pass through the plate **2** from an upper surface **23** to a bottom surface **25.** Thus, electromagnetic radiation directed to the plate will transverse the plate only at the apertures **22.** The apertures **22** are arranged in a circular array so as to overly the sclera limbus of an eye being treated. The circular array of apertures may have a diameter in the range from 11 to 13 mm, which is the typical diameter of the sclera limbus. The plate 21 may have as many as 200 apertures equally spaced along the circumference of the disk 21, so that 200 spots in the sclera limbus can be treated simultaneously.

This is where the reduced-energy aiming beam comes into play. It should be aimed as to cover the whole circumference of the limbus. When the operator sees the aiming beam positioned properly, he activates the treatment beam. See first para on page 5 above.

Fig. 3 shows a device **24** for directing electric magnetic radiation to one or more regions of a limbal area of an eye in accordance with another embodiment of the invention. The device **24** comprises a thin plate **26** that may be a circular disk. The plate **2** is provided with a plurality of small apertures **28** having the shape of circular arcs. The plate **26** is formed from an opaque material such as metal. The apertures **28** pass through the plate **26** from an upper surface **27** to a bottom surface **29.** Thus, electromagnetic radiation directed to the plate **26** will transverse the plate only at the apertures **28.** The apertures **28** are arranged in a circular array so as to overly the sclera limbus of an eye being treated. The circular array of apertures may have a diameter in the range from 11 to 13 mm, which is the typical diameter of the sclera limbus.

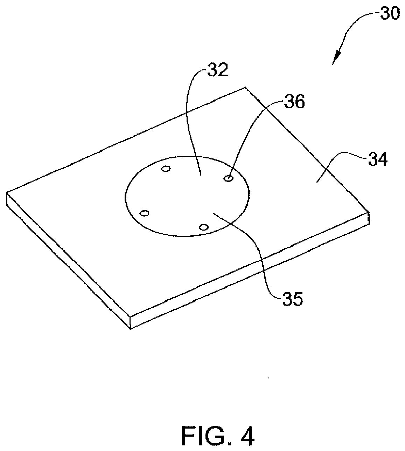

Fig. 4 shows a device **30** for directing electric magnetic radiation to one or more regions of a limbal area of an eye in accordance with yet another embodiment of the invention. The device **30** comprises a circular disk **32** that rotates in a circular hole in a thin plate **34.** The disk **32** is provided with a plurality of small apertures **36** that may have a circular cross section. The disk **32** is formed from an opaque material such as metal. The apertures **36** pass through the disk **32** from an upper surface **35** to a bottom surface. Thus, electromagnetic radiation directed to the circular disk **32** will transverse the plate only at the apertures **36.** The apertures **36** are arranged a long a circle to overly the sclera limbus of an eye being treated. The circular array of apertures may have a diameter in the range from 11 to 13 mm, which is the typical diameter of the sclera limbus. In use, the disk **32** may be rotated between pulses of electric magnetic radiation to deliver the radiation to a number of spots in the sclera limbus that is significantly greater than the number of apertures in the disk.

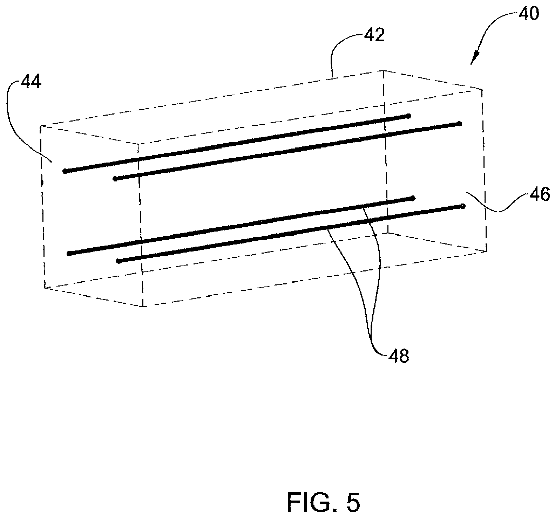

Fig. 5 shows a device **40** for directing electric magnetic radiation to one or more regions of a limbal area of an eye in accordance with still another embodiment of the invention. The device **40** comprises a block 42 of an opaque material shown in phantom drawing in Fig. 5. The block **32** has a first face **44** and an oppositely situated second face **46.** One or more optic fibers **48** extend from the first face **44** to the second face **46.** Thus, electromagnetic radiation directed to the first face **44** will transverse the block **42** only along the optic fibers **48.** The optic fibers **48** are arranged in a cylinder so that the ends of the optic fibers **48** in the second face **46** overly the sclera limbus of an eye being treated. The cylinder of optic fibers **48** may have a diameter in the range from **11** to **13** mm, which is the typical diameter of the sclera limbus.

In another embodiment of the device of the invention, the device comprises a refractive or diffractive optical element. The refractive or diffractive optical element may be made from glass or plastic having transmitting and refracting or diffractive optics which will create a circular beam or rapidly deliver a number of discrete beams to the limbal area. When electromagnetic radiation is incident on the refractive or optical element, the radiation exits the opposite side of the element as a beam having an annular cross section. This allows radiation of a complete circle around the limbal area by a continuous ring of light. The annulus of light may have, for example, a diameter between 9 and 13 mm, and may be from 0.5 to 2.5 mm in radial width. The lasers involve may be doubled Nd/YAG, argon or any diode emitting radiation in the visible or infra red

In another embodiment of the device of the invention, the optical device includes an ellipsoidal or parabolic mirror that when illuminated by a large spot of light scanning along a large circle will generate a small ring at its focal plane.

The optical device may be a lens. The lens may illuminate a single point of the limbal area, in which case, the system may include a manipulator to allow the laser beam to be directed to a plurality of locations around the limbal area in succession to impact on a plurality of locations of the trabecular meshwork. A first point around the limbal area can be illuminated, after which, the laser beam can be directed towards a second point around the limbus, and so on. This can be done automatically and rapidly. Up to about 200 points can be illuminated simultaneously at the treatment intensity with a single laser.

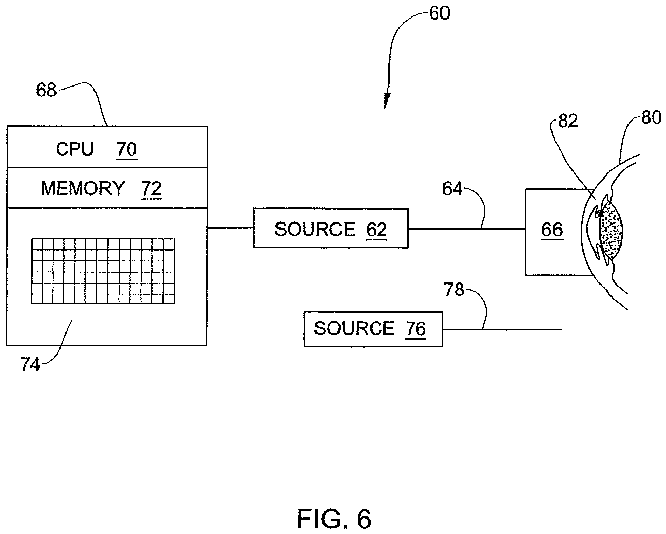

Turning now to Fig. 6, a system **60** is shown schematically for treating an eye in accordance with one embodiment of this aspect of the invention. The system **60** comprises a source **62** of electromagnetic radiation that generates a beam of electromagnetic radiation **64.** The system **60** also includes a device **66** for directing the beam **64** radiation to one or more regions of a limbal area of an eye. The device **66** may be, for example, any one of the devices **20, 24, 30** or **40** described above. Operation of the source **62** is under the control of a processing unit **68** which comprises a CPU **70,** a memory **72** and a user input device, such as a keypad **74.**

The beam **64** can have a wavelength, for example, between 514 and 810 nm. The source **62** may be a laser in the visible or near infrared range, such as a 532 Nd:YAG laser.

The user input device **74** may be used to input parameters relating to the treatment. For example, a user may input a beam intensity, a number of pulses of electromagnetic radiation that is to be delivered to the eye, and a pulse rate. The parameters may be stored in the memory **72.** The memory may also be used to store data relating to the individual being treated, as well as any relevant observations relating to the treatment.

The pulses may be between 0.1 to 3 nanoseconds, and the fluence of a single pulse may be 0.84 to 10,000 J/cm². The total energy delivered to a single eye may be from 4 to 20 J. At this fluence, the beam **64** is not visible. The system **60** may thus include a second source **76** of electromagnetic radiation that produces a visible light beam **78.** The source **76** may be temporarily positioned to direct the beam **78** to the device **66** in order to properly position the device **66** over the eye **80** to be treated. The device **66** is properly positioned over the eye when the beam **64** or the beam **78** impinging on the device 66 is delivered only to the limbal area **82** of the eye **80.**

In use, the device **66** is positioned over the eye **80.** As stated above, the device **66** is properly positioned over the eye when the beam **64** is delivered only to the limbal area **82** of the eye **80.** The source **62** is then activated to generate a predetermined sequence of one or more pulses of the beam **64.** At any time, the device 66 may be rotated over the eye 80 and another sequence of one or more pulses may be generated. The process may be repeated as required in any treatment.

The following numbered clauses on pages 13 to 16 of the present description correspond to the claims of European patent application no. 11724805.4 as filed. The claims of the present application as filed, which is divided from European patent application no. 11724805.4, can be found on the subsequent pages 17 and 18 of the specification which begin with the heading "CLAIMS".

### CLAUSES:

1. A device for delivering electromagnetic radiation to a limbal area of an eye, the device having a first side and a second side, and the device transforming one or more beams of electromagnetic radiation that are incident on the first side into one or more beams of the electromagnetic radiation that are emitted from the second side, wherein the one or more emitted beams are arrayed in an array selected from:
   **(a)** an array of two or more emitted beams that are arrayed in a cylindrical array, the cylindrical array having a circular cross section;
   **(b)** an array of one or more beams each beam having a cross sectional shape of a circular arc; and
   **(c)** an array comprising a beam having an annular cross section.
2. The device according to clause 1 wherein the emitted radiation comprises two or more emitted beams that are arrayed in a cylindrical array, the cylindrical array having a circular cross section, the cross section having a diameter from 9 to 13 mm.
3. The device according to clause 1 wherein the emitted radiation comprises an array of one or more beams having a cross sectional shape of a circular arc; the circular arc having a diameter from 9 to 13 mm.
4. The device according to clause 1 wherein the emitted radiation comprises an array comprising a beam having an annular cross section, the annular cross section having an inner diameter and an outer diameter, the average of the inner diameter and the outer diameter being from 9 to 13 mm.
5. The device according to clause 1 wherein the emitted radiation comprises two or more emitted beams that are arrayed in a cylindrical array, the device comprising an element opaque to the electromagnetic radiation having therein a circular array of apertures extending from a first face of the opaque element to a second face of the opaque element.
6. The device according to clause 5 wherein the circular array of apertures comprises at least 50 apertures
7. The device according to clause 5 or 6 wherein the circular array of apertures has a center and the opaque element is rotatable about the center of the circular array.
8. The device according to clause 1 wherein the emitted radiation comprises an array of one or more beams having a cross sectional shape of a circular arc; the device comprising an element opaque to the electromagnetic radiation having therein a circular array of apertures, each aperture having a cross sectional shape of a circular arc and each aperture extending from a first face of the opaque element to a second face of the opaque element.
9. The device according to clause 8 wherein the circular array of apertures has a center and the opaque element is rotatable about the center of the circular array.
10. The device according to clause 1 wherein the emitted radiation comprises two or more emitted beams that are arrayed in a cylindrical array, the device comprising a cylindrical array of optic fibers.
11. The device according to clause 10 wherein the cylindrical array of optic fibers is embedded in an opaque element, and the cylindrical array of optic fibers extends from a first face of the opaque element to a second face of the opaque element.
12. The device according to clause 1 wherein the emitted radiation comprises a beam having an annular cross section, and the device comprises a refractive or diffractive optical element.
13. The device according to clause 1 wherein the emitted radiation comprises a beam having an annular cross section, and the device comprises an ellipsoidal mirror.
14. The device according to any one of the previous clauses further comprising concave a surface adjacent to the second side of the device.
15. An ensemble comprising two or more devices for delivering electromagnetic radiation from the first source of electromagnetic radiation to a limbal area of an eye, each of the one or more devices having a first side and a second side, and each device transforming one or more beams of electromagnetic radiation that are incident on the first side into one or more beams of the electromagnetic radiation that are emitted from the second side, wherein the one or more emitted beams are arrayed in an array selected from:
   (i) an array of two or more emitted beams that are arrayed in a cylindrical array, the cylindrical array having a circular cross section, wherein each of the one or more devices producing a cylindrical array of emitted beams produces a cylindrical array of emitted beams having a different cross-sectional diameter;
   (ii) an array of one or more beams having a cross sectional shape of a circular arc wherein each of the one or more devices producing emitted beams having a cross sectional shape of a circular arc produces emitted beams having a cross sectional shape of a circular arc of a different diameter ; and
   (iii) an array comprising a beam having an annular cross section, wherein each of the one or more devices producing an emitted beam having an annular cross section produces an emitted beam having an annular cross section of one or both of a different inner diameter or a different outer diameter.
16. A system for delivering electromagnetic radiation to a limbal area of an eye, comprising:
   **(a)** a first source of electromagnetic radiation; and
   **(b)** one or more devices for delivering electromagnetic radiation from the first source of electromagnetic radiation to a limbal area of an eye the device when the first source is optically coupled to the device, each of the one or more devices having a first side and a second side, and each device transforming one or more beams of electromagnetic radiation from the first source that are incident on the first side into one or more beams of the electromagnetic radiation that are emitted from the second side, wherein the one or more emitted beams are arrayed in an array selected from:
      (i) an array of two or more emitted beams that are arrayed in a cylindrical array, the cylindrical array having a circular cross section, wherein each of the one or more devices produces a cylindrical array of emitted beams having a different cross-sectional diameter;
      (ii) an array of one or more beams having a cross sectional shape of a circular arc wherein each of the one or more devices produces emitted beams having a cross sectional shape of a circular arc of a different diameter; and
      (iii) an array comprising a beam having an annular cross section, wherein each of the one or more devices produces emitted beams having an annular cross section of a different inner diameter or a different outer diameter.
17. The system according to clause 16 wherein the first source is a laser.
**18.** The system according to clause 17 wherein the laser has a wavelength from 514 810 nm.
19. The system according to clause 18 wherein the laser is a 532 Nd:YAG laser.
20. The system according to any one of clauses 16 to 19 further comprising a processor configured to execute a predetermined regime of activation of the first source of electromagnetic radiation.
21. The system according to clause 20 wherein the predetermined regime of activation of the source of electromagnetic radiation comprises a series of pulses.
22. The system according to clause 21 wherein the pulses are between 0.1 to 3 nanoseconds in duration.
23. The system according to clause 21 or 22 wherein the fluence of a single pulse is from 0.84 to 10,000 J/cm².
24. The system according to any one of clauses 21 to 23 wherein the total energy delivered to a single eye is from 4 to 20 J.
25. The system according to any one of clauses 16 to 24 further comprising a second source of electromagnetic radiation that produces a visible light beam, the second source of radiation being configured to be optically coupled to the device of the system.
26. A method for delivering electromagnetic radiation to a limbal area of an eye, comprising
   **(a)** delivering electromagnetic radiation to the limbal area of the eye wherein the delivered electromagnetic radiation is in the form of one or more beams, the beams being arrayed in an array selected from:
      (i) an array of two or more emitted beams that are arrayed in a cylindrical array, the cylindrical array having a circular cross section;
      (ii) an array of one or more beams having a cross sectional shape of a circular arc; and
      (iii) an array comprising a beam having an annular cross section.
27. The method according to clause 26 for use in the treatment of glaucoma.

## Claims

1. Apparatus, comprising:
a laser source, which is configured to generate electromagnetic radiation; and
a device which is configured to irradiate through the limbal area one or more regions of a trabecular meshwork of an eye with the electromagnetic radiation, by directing the electromagnetic radiation to a sclera limbus of the eye without making contact between the apparatus and the eye.

2. The apparatus according to claim 1, wherein, by irradiating the trabecular meshwork with the electromagnetic radiation, the laser source and the device are configured to treat glaucoma in the eye.

3. The apparatus according to claim 1, wherein, by irradiating the trabecular meshwork with the electromagnetic radiation, the laser source and the device are configured to treat narrow-angle or closed-angle glaucoma in the eye.

4. The apparatus according to claim 1, wherein the device is configured to direct the electromagnetic radiation to the eye not through a gonioscopic lens.

5. The apparatus according to claim 1, wherein the device is configured to direct the electromagnetic radiation, simultaneously or sequentially, to multiple points distributed on a scleral side of a limbus of the eye.

6. The apparatus according to claim 1, wherein each of the regions is arch-shaped.

7. The apparatus according to claim 1, wherein each of the regions is circular.

8. The apparatus according to claim 1, wherein the device comprises a scanning mirror.

9. The apparatus according to claim 1, wherein the device comprises an array of optical fibers whose ends are aimed toward multiple regions of the trabecular meshwork, without making contact with the eye.

10. The apparatus according to claim 1, wherein the device comprises a refractive or diffractive optical element.

11. The apparatus according to claim 1, wherein the laser source is further configured to generate a visible aiming beam for aiming the electromagnetic radiation by an operator, and wherein the device is configured to direct both the electromagnetic radiation and the aiming beam.

12. Apparatus according to claim 1, in which the trabecular meshwork is irradiated directly through the limbal area.

13. Apparatus according to claim 1 or 2, in which the trabecular meshwork is irradiated using a laser of wavelength between 514 and 810nm so as to penetrate a limbal area of up to 1mm in thickness
